# EUROPÄISCHE PATENTANMELDUNG

(11) **EP 2 052 701 A1**
(43) Veröffentlichungstag der Anmeldung: **29.04.2009**
(21) Anmeldenummer: 08166199.3
(22) Anmeldetag: 09.10.2008
(51) Int. Cl.: A61F 2/90

(54) **Stent aus Nitinol mit verbesserter axialer Biegesteifigkeit und dazugehöriges Herstellungsverfahren**

(30) Priorität: 24.10.2007 DE 102007050666
(71) Anmelder: BIOTRONIK VI Patent AG, 6341 Baar (CH)
(72) Erfinder: Lootz, Daniel, 18119, Rostock (DE); Block, Bernd, 18055, Rostock (DE)
(74) Vertreter: Lindner-Vogt, Karin L.

(57) **Zusammenfassung**

Die Erfindung betrifft einen Stent (20) aus Nitinol mit verbesserter axialer beziehungsweise radialer Steifigkeit, wobei der Stent eine Tragstruktur aufweist, die in Umfangsrichtung umlaufende Stege umfasst, die in axialer Richtung über Verbindungsstege (22) miteinander verknüpft sind. Die Tragstruktur kann einen ersten komprimierten Zustand und einen zweiten expandierten Zustand einnehmen. Das Nitinol liegt in der Tragstruktur im ersten komprimierten Zustand in einem martensitischen Gefüge und im zweiten expandierten Zustand weitgehend in einem austenitischen Gefüge vor. Ein oder mehrere Tragstrukturabschnitte liegen jedoch ganz oder in Teilen im zweiten expandierten Zustand in einem martensitischen Gefüge vor.

## Beschreibung

Die Erfindung betrifft einen Stent aus Nitinol mit adaptierter axialer bzw. radialer Steifigkeit, wobei der Stent eine Tragstruktur aufweist, die in Umfangsrichtung umlaufende Stege umfasst, die in axialer Richtung über Verbindungsstege miteinander verknüpft sind. Die Erfindung betrifft weiterhin ein dazugehöriges Herstellungsverfahren für den Stent.

### Technologischer Hintergrund und Stand der Technik

Nitinol ist eine Nickel-Titan-Legierung und wohl der bekannteste Vertreter der Formgedächtnis-Legierungen. Nitinol weist eine kubische Kristallstruktur auf, die zu ca. 55 wt% aus Nickel und dem Rest Titan besteht. Die Legierung ist bis 650°C verwendbar, korrosionsbeständig und hochfest, dabei jedoch bis ca. 8% pseudoelastisch verformbar.

Voraussetzung für den Formgedächtnis-Effekt ist eine sogenannte martensitische Umwandlung, bei der die beteiligten Phasen, Hochtemperaturphase (Austenit) und Niedertemperaturphase (Martensit), geordnete Gitterstrukturen aufweisen. Bei der Umwandlung in Formgedächtnislegierungen treten nur sehr geringe elastische Spannungen auf. Durch Bildung sogenannter Zwillinge in speziell orientierten Martensitplatten wird die durch Versetzungsbewegung verursachte irreversible plastische Verformung nahezu vollständig vermieden. Der kubisch raumzentrierte Austenit wandelt sich bei Abkühlung in eine verzwillingte Martensitstruktur um. Diese Umwandlung erfolgt diffusionslos, unter Wärmeabgabe, durch Klappvorgänge und ist nicht mit einer Änderung der Form des Objektes verbunden. Der Martensit ist durch Entzwillingen leicht verformbar und diese Verformung ist bleibend, solange das Material unter der Umwandlungstemperatur verbleibt. Wird der verformte Martensit jedoch erwärmt, stellt sich bei Überschreiten der Umwandlungstemperatur die ursprüngliche Kristallorientierung der Hochtemperaturphase und damit die ursprüngliche Gestalt wieder ein. Die Umwandlung Austenit/Martensit und die Rückumwandlung Martensit/Austenit finden bei unterschiedlichen Temperaturen statt.

Die beiden Phasen zeigen charakteristische Unterschiede im Festigkeitsverhalten. Das Verfestigungsverhalten des Martensits ist recht ungewöhnlich. Es ist gekennzeichnet durch das sogenannte Martensitplateau, einen Bereich mit sehr geringer Verfestigung. Hier findet die Verformung durch Entzwillingen statt. Wenn diese Verformungsmöglichkeit erschöpft ist (nach ca. 8% Dehnung), müssen andere Verformungsarten aktiviert werden. An das Martensitplateau schließt sich somit ein zweiter elastischer Bereich an. Bei Erreichen der wahren Streckgrenze erfolgt die Verformung konventionell durch Versetzungsbewegung. Die Verformung im Bereich des Martensitplateaus kann durch Erwärmung rückgängig gemacht werden. Ein ebenfalls ungewöhnliches Spannungs-Dehnungs-Diagramm wird bei der Verformung des Austenits unterhalb einer Grenztemperatur für die spannungsinduzierte Martensitbildung beobachtet. Hier tritt der Effekt der sogenannten Pseudo- oder Superelastizität auf, der auf die Bildung von spannungsinduziertem Martensit zurückzuführen ist.

Die Martensitbildung kann nicht nur durch thermische sondern auch durch mechanische Triebkräfte bewirkt werden. Wird eine Formgedächtnislegierung im Hochtemperaturzustand belastet, werden Martensitzwillinge induziert, die sofort entzwillingen. Mit zunehmender Spannung wird mehr Martensit induziert und entzwillingt. Das so entstandene Martensit ist jedoch thermisch instabil, d.h. bei Wegfall der äußeren Spannungen findet sofort eine Rückumwandlung statt. Die Probe nimmt ihre ursprüngliche Gestalt wieder an. Bei Temperaturen oberhalb einer bestimmten Grenztemperatur kann kein Martensit mehr induziert werden. Es ist nun leichter, Versetzungen zu erzeugen und zu bewegen. Das Spannungs-/Dehnungs-Diagramm des Austenits gleicht dann dem konventioneller Legierungen.

Je nach vorangegangener thermomechanischer Behandlung lassen sich damit drei mögliche Effekte unterscheiden: Beim Einwegeffekt wird eine große, bleibende Verformung durch Erwärmen um wenige Grad Celsius rückgängig gemacht. Beim Zweiwegeffekt erinnert sich das Bauteil aus der Formgedächtnislegierung an zwei vorher eingeprägte, unterschiedliche Formen und nimmt diese bei Erwärmen und Abkühlen abwechselnd ein. Die Pseudoelastizität bewirkt schließlich ein elastisches Verhalten, wobei fast konstante Kräfte über große Verformungswege ausgeübt werden.

Formgedächtnislegierungen, insbesondere eben Nitinol, finden in der Medizintechnik unter anderem Anwendung in Form von selbstexpandierenden Stents. Ein Stent ist ein medizinisches Implantat, das in bestimmte Organe eingebracht wird, um ihre Wand ringsum abzustützen. Beim Nitinol-Stent handelt es sich um eine aus Nitinol bestehende kleine Tragstruktur in Röhrchenform, die einen komprimierten Zustand mit geringem Durchmesser und einen expandierten Zustand mit für den Bestimmungszweck vorgebbaren vergrößerten Durchmesser einnehmen kann.

Verwendung finden Stents zum einen in Blutgefäßen, speziell den Herzkranzgefäßen, um nach deren Aufdehnung (PTCA) einen erneuten Verschluss zu verhindern; eine solche Behandlung wird als Stentangioplastie bezeichnet. Zum anderen dienen Stents in der Krebsbehandlung dazu, durch bösartige Tumore verursachte Verengungen von, zum Beispiel der Atemwege, Gallenwege oder der Speiseröhre nach einer Aufdehnung offen zu halten.

Der Nitinol-Stent wird im komprimierten Zustand durch einen äußeren Polymerschlauch, der Schutzhülle, auf einem Kathetersystem fixiert und so an den Implantationsort verbracht. Der Stent wird nun durch das Zurückziehen des äußeren Polymerschlauches freigesetzt. Durch das pseudoelastische Verhalten der Nickel-Titan-Legierung öffnet sich der Stent von selbst und stützt die Gefäßwand/Organwand ab.

Am Markt befindliche selbstexpandierende Nitinol-Stents lassen sind generell hinsichtlich ihres Designs, speziell ihres Vernetzungsgrades, in zwei Kategorien unterteilen, nämlich in offenzellige und geschlossenzellige Designs.

Offenzellige Designs sind sehr flexibel konstruierbar, haben aber den Nachteil, dass sie ohne Verwendung zusätzlicher Hilfsmittel nicht in die Schutzhülle des Katheters reponierbar sind. Ursache ist ihre geringe Vernetzung in axialer Richtung.

Insbesondere geschlossenzellige Designs besitzen eine Tragstruktur, die in Umfangsrichtung umlaufende Stege umfasst, die in axialer Richtung über Verbindungsstege miteinander verknüpft sind. Aufgrund des hohen axialen Vernetzungsgrads über die Verbindungsstege können geschlossenzellige Designs nach partieller Freisetzung wieder in die Schutzhülle des Kathetersystems zurückgezogen werden (sogenannte Reponierbarkeit). Der hohe Vernetzungsgrad durch die axialen Verbindungsstege sorgt jedoch für eine wesentlich höhere Biegesteifigkeit im Vergleich zum offenzellig konstruierten Stent. Für Stents aus Nitinol mit einem geschlossenzelligen Design besteht daher das Bedürfnis die axiale Flexibilität des Stents zu erhöhen ohne jedoch die Reponierbarkeit einzuschränken.

### Erfindungsgemäße Lösung

Ein erster Aspekt der Erfindung liegt in der Bereitstellung eines Stents aus Nitinol mit einer Tragstruktur, die in Umfangsrichtung umlaufende Stege umfasst, die in axialer Richtung über Verbindungsstege miteinander verknüpft sind. Die Tragstruktur kann einen komprimierten Zustand und einen expandierten Zustand einnehmen, wobei das Nitinol in der Tragstruktur im komprimierten Zustand in einem martensitischen Gefüge und im expandierten Zustand weitgehend in einem austenitischem Gefüge vorliegt. Erfindungsgemäß liegen jedoch eine oder mehrere Tragstrukturabschnitte, insbesondere die Verbindungsstege, ganz oder in Teilen im expandierten Zustand in einem martensitischen Gefüge vor. Aufgrund der geringeren Reaktionskräfte bei vergleichbaren Dehnungen ist bei Verwendung der Martensitstruktur (vgl. mit Austenit) eine weitere Steifigkeitsreduktion in axialer beziehungsweise radialer Erstreckung des Stents denkbar.

Durch die erfindungsgemäße Lösung können die Nachteile eines geschlossenzelligen Stentdesigns hinsichtlich der hohen axialen Biegesteifigkeit oder auch radialen Steifigkeit gelöst oder zumindest gemindert werden. Die Verbesserung ist durch das zirka halb so große E-Modul und die geringere Festigkeit des Martensits gegenüber dem Austenit zu erklären. Ein individuelles Stentdesign weist demnach einen oder mehrere Tragstrukturabschnitte, insbesondere Verbindungsstege auf, die ganz oder nur in Teilabschnitten im expandierten Zustand des Stents aus Nitinol mit martensitischem Gefüge bestehen. Die Anzahl der erfindungsgemäß zu modifizierenden Tragstrukturabschnitte sowie der Umfang der Modifikation im Bereich der Tragstrukturabschnitte hängen vom konkreten Stentdesign und dem angestrebten Einsatzzweck des Stents ab. Es versteht sich, dass eine Gefügeänderung im Sinne der Erfindung über die gesamte Länge zum Beispiel aller Verbindungsstege einen maximalen reduzierenden Effekt auf die axiale Biegesteifigkeit des Stents hat. Der Fachmann hat nun zum Beispiel die Möglichkeit, die Gefügemodifikation im Bereich der Verbindungsstege auch in einem geringeren Umfang zu forcieren und damit einen Stent zu schaffen, dessen axiale Biegesteifigkeit zwischen den genannten beiden Extremen des Ausgangszustands und einem Stent mit vollständig im Gefüge umgewandelten Verbindungsstegen liegt.

Des Weiteren besteht die Möglichkeit ganz oder in Teilen martensitische Tragstrukturabschnitte entlang der Längsachse eines Stents zu schaffen. Dadurch ist eine adaptierte radiale Steifigkeit des Stents an die Erfordernisse des Implantationsortes denkbar. Die in Umfangsrichtung umlaufenden Stege weisen vorzugsweise eine mäander- aber auch spiralförmige Kontur auf.

Vorzugsweise weisen die Verbindungsstege eine V-, W- oder S-förmig Kontur auf. Derartige Verbindungsstege erlauben Stentdesigns mit einer sehr gleichmäßigen Abstützung der Gefäßwand/des Organs im expandierten Zustand des Stents, haben jedoch den Nachteil, dass sie eine besonders hohe axiale Steifigkeit besitzen. Durch die erfindungsgemäße Modifikation des Gefüges im Bereich der Verbindungsstege kann die axiale Biegesteifigkeit jedoch wieder auf ein vertretbares Maß reduziert werden. Die Gefügemodifikation im Bereich der Verbindungsstege erfolgt vorzugsweise im Bereich von Wendepunkten (im Sinne Ihrer mathematischen Definition) der V, W- oder S-förmige Kontur, so dass diese im expandierten Zustand in einem martensitischen Gefüge vorliegen.
Eine weitere bevorzugte Ausführungsform - die sich auch in Kombination mit den zuvor geschilderten V-, W- und S-förmigen Konturen der Verbindungsstege realisieren lässt - sieht vor, dass die in Umfangsrichtung umlaufenden Stege eine mäanderförmige Kontur besitzen. Mit anderen Worten, die Stege laufen wellenförmig mit vorgegebener Amplitude um eine Längsachse des Stents. Ein solches Design ermöglicht eine besonders gleichmäßige Abstützung des umgebenden Gewebes im expandierten Zustand des Stents.

Ein weiterer Aspekt der Erfindung liegt in der Bereitstellung eines Verfahrens zur Herstellung eines Stents aus Nitinol mit einer Tragstruktur, die in Umfangsrichtung umlaufende Stege umfasst, die in axialer Richtung über Verbindungsstege miteinander verknüpft sind. Die Tragstruktur kann einen komprimierten Zustand und einen expandierten Zustand einnehmen, wobei das Nitinol in der Tragstruktur im komprimierten Zustand in einem martensitischen Gefüge und im expandierten Zustand weitgehend in einem austenitischen Gefüge vorliegt. Ein oder mehrere Tragstrukturabschnitte, insbesondere Verbindungsstege, liegen jedoch ganz oder in Teilen im expandierten Zustand in einem martensitischen Gefüge vor. Zur Herstellung eines derart modifizierten Stents umfasst das Verfahren die Schritte:
(i) Bereitstellen eines Stents, dessen Tragstruktur im expandierten Zustand vollständig in einem austenitischen Gefüge vorliegt; und
(ii) teilweises oder vollständiges Erwärmen ein oder mehrerer Tragstrukturabschnitte der Tragstruktur über eine Temperatur von 200°C, um die Martensitbildung zu induzieren.

Vorzugsweise sind die Tragstrukturabschnitte Verbindungsstege und diese werden im Schritt (ii) teilweise oder vollständig auf über 200°C erwärmt.

Ein hierzu alternatives Verfahren zur Herstellung eines in eben gleicher Weise modifizierten Stents umfasst die Schritte:
(i) Bereitstellen eines Rohrs, das vollständig in einem austenitischen Gefüge vorliegt;
(ii) teilweises oder vollständiges Erwärmen ein oder mehrerer Rohrabschnitte über eine Temperatur von 200°C, um die Martensitbildung zu induzieren;
(iii) Herausschneiden der Tragstruktur des Stents aus dem Rohr.

Demgemäß wird nach dieser Herstellungsvariante ein Rohr aus Nitinol einer lokalen thermischen Behandlung unterzogen. Aus dem Rohr wird anschließend in bekannter Weise die Tragstruktur des Stents geschnitten.

Durch die lokale Wärmebehandlung, d. h. das Erwärmen der zu behandelnden Bereiche der Verbindungsstege, Tragstrukturen oder Rohrabschnitte auf eine Temperatur oberhalb von 200°C, wird austenitisches Gefüge von Nitinol in ein martensitisches oder teilmartensitisches Gefüge umgewandelt. Durch die lokale Wärmebehandlung soll erreicht werden, dass die Umwandlungstemperatur (Af - austenite finish) im Stent lokal unterschiedlich ist. Dadurch liegen bei beispielsweise konstanter Umgebungstemperatur von 37°C lokal unterschiedliche Gefügezustände im Stent vor. Sind martensitische/teilmarten-sitische Bereiche in den Verbindungsstegen, so ist mit einer geringeren Biegesteifigkeit zu rechnen, verglichen mit einem Stent, der vollständig aus austenitischem Gefüge besteht. Sind teilmartensitische oder martensitische Bereiche in der Tragstruktur (beispielsweise abwechselnd zu benachbarten Tragstrukturen) so ist mit einem unterschiedlichen radialen Steifigkeitsverhalten entlang der Stentlängsachse zu rechnen. In beiden Fällen werden die mechanischen Eigenschaften des Martensits mit seinem geringeren Festigkeitsverhalten verglichen mit Austenit genutzt.

Für die lokale oder abschnittsweise Wärmebehandlung können energetische Verfahren, die thermische Effekte erzielen, verwendet werden. Vorzugsweise erfolgt das Erwärmen im Schritt (ii) beider Verfahren mittels Laser- oder Elektronenstrahl, mittels Plasma oder induktiv. Die genannten Methoden erlauben eine lokale Gefügeumwandlung in sehr kleinen oder flächigen Dimensionen und sind daher besonders geeignet für filigrane Strukturen von Stents oder an Rohren.

Nachfolgend wird die Erfindung anhand von Ausführungsbeispielen und der dazugehörigen Zeichnungen näher erläutert. Es zeigen:
- Fig. 1 und 2: Zwei Beispiele für Rohre aus Nitinol mit abschnittweise wechselnder Radialsteifigkeit;
- Fig. 3: einen Ausschnitt aus einer Tragstruktur eines Stents mit erfindungsgemäß modifizierten Verbindungsstegen; und
- Fig. 4 bis 6: Beispiele für S-, V- und W-förmige Verbindungsstege mit modifizierter axialer Biegefestigfestigkeit.

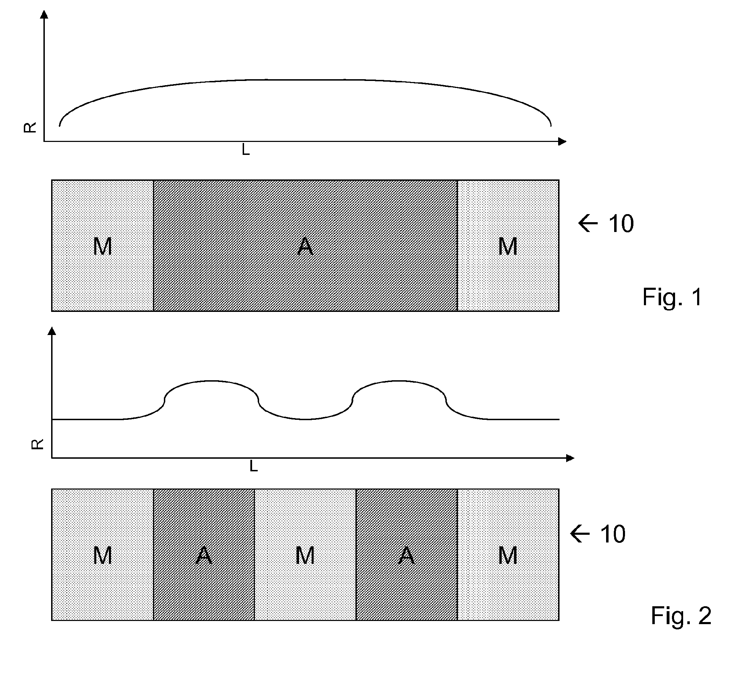

Den Figuren 1 und 2 sind jeweils stark schematisiert Rohre 10 aus Nitinol zu entnehmen, die abschnittsweise im Sinne der Erfindung thermisch behandelt wurden, um ihre Radialsteifigkeit R über die Länge L der Rohre 10 zu modifizieren. Hierzu wird zunächst von einem vollständig in einem austenitischen Gefüge vorliegendem Rohr 10 ausgegangen. In ausgewählten Rohrabschnitten erfolgt nun eine thermische Behandlung durch Erwärmen auf Temperaturen von über 200°C, beispielsweise mittels eines Lasers. In der Folge gehen die so behandelten Rohrabschnitte in ein martensitisches Gefüge über.

In den Figuren 1 und 2 sind Rohrabschnitte aus Martensit mit M und Rohrabschnitte aus Austenit mit A gekennzeichnet. Weiterhin ist schematisch die Abhängigkeit der Radialsteifigkeit R über die Rohrlänge L dargestellt. Dem entsprechend wird ein aus einem derart modifizierten Rohr 10 hergestellter Stent in verschiedenen Abschnitten seiner Tragstruktur eine unterschiedliche Radialfestigkeit und Biegesteifigkeit schon aufgrund der unterschiedlich vorliegenden Gefüge von Nitinol besitzen.

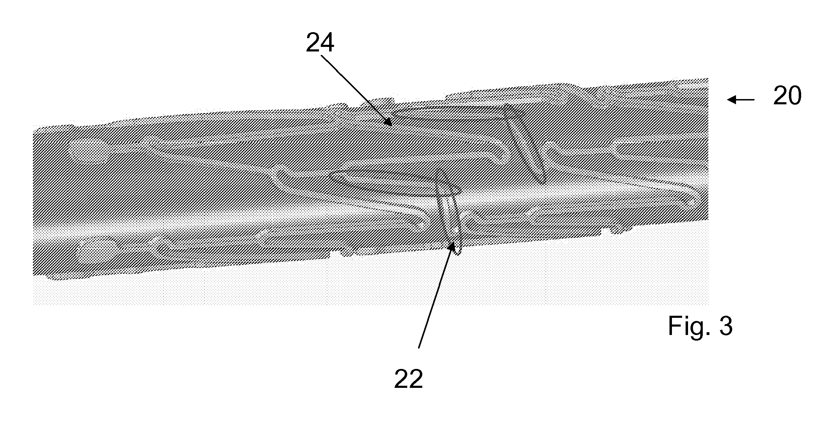

Figur 3 ist ein Ausschnitt aus einer Tragstruktur eines Stents 20 mit erfindungsgemäß modifizierten Verbindungsstegen 22 zu entnehmen. Die umrandeten Strukturbereiche markieren die axialen Verbindungsstege 22, welche radial tragende Strukturbereiche 24 - hier als mäandrierende Helix ausgeführt - untereinander verbinden. Die Verbindungsstege 22 bestimmen maßgeblich das mechanische Verhalten des Stens 20 unter Biegebelastung.

Erfindungsgemäß werden die Verbindungsstege 22 teilweise oder ganz derart modifiziert, dass sie bei Körpertemperatur im martensitischen Gefügezustand vorliegen, während sich die restlichen Strukturbereiche 24 im austenitischen Zustand befinden. In der Folge ist die axiale Biegesteifigkeit des Implantats reduziert.

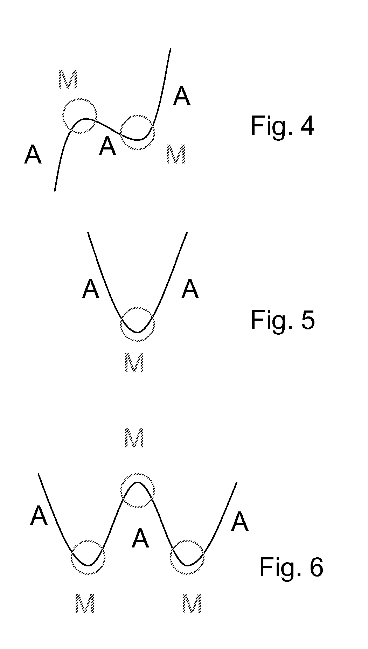

Den Figuren 4 bis 6 sind stark schematisiert Verbindungsstege mit S-, V- und W-förmigen Konturen zu entnehmen, die jeweils im Bereich ihrer Wendepunkte durch lokale thermische Behandlung derart modifiziert sind, dass bei Körpertemperatur ein martensitischer Gefügezustand vorliegt, während in den verbleibenden Abschnitten der Verbindungsstege nach wie vor ein austenitischer Gefügezustand vorliegt.

## Patentansprüche

1. Stent (20) aus Nitinol mit einer Tragstruktur, die in Umfangsrichtung umlaufende Stege umfasst, die in axialer Richtung über Verbindungsstege (22) miteinander verknüpft sind, und bei der die Tragstruktur einen komprimierten Zustand und einen expandierten Zustand einnehmen kann, wobei das Nitinol in der Tragstruktur im komprimierten Zustand in einem martensitischen Gefüge und im expandierten Zustand weitgehend in einem austenitischen Gefüge vorliegt, jedoch ein oder mehrere Tragstrukturabschnitte ganz oder in Teilen im expandierten Zustand in einem martensitischen Gefüge vorliegen, wobei eine Martensitbildung durch teilweises oder vollständiges Erwärmen eines oder mehrerer Tragstrukturabschnitte über eine Temperatur von 200°C induziert wurde.

2. Stent nach Anspruch 1, bei dem die in Umfangsrichtung umlaufenden Stege eine mäanderförmige Kontur aufweisen.

3. Stent nach Anspruch 1 oder 2, bei dem ein oder mehrere Verbindungsstege (22) ganz oder in Teilen im expandierten Zustand in einem martensitischen Gefüge vorliegen.

4. Stent nach Anspruch 3, bei dem die Verbindungsstege (22) eine V-, W- oder S-förmige Kontur aufweisen.

5. Stent nach Anspruch 4, bei dem die Verbindungsstege (22) im Bereich von Wendepunkten der V-, W- oder S-förmige Kontur im expandierten Zustand in einem martensitischen Gefüge vorliegen.

6. Verfahren zur Herstellung eines Stents aus Nitinol mit einer Tragstruktur, die in Umfangsrichtung umlaufende Stege umfasst, die in axialer Richtung über Verbindungsstege miteinander verknüpft sind, und bei der die Tragstruktur einen komprimierten Zustand und einen expandierten Zustand einnehmen kann, wobei das Nitinol in der Tragstruktur im komprimierten Zustand in einem martensitischen Gefüge und im expandierten Zustand weitgehend in einem austenitischen Gefüge vorliegt, jedoch ein oder mehrere Tragstrukturabschnitte ganz oder in Teilen im expandierten Zustand in einem martensitischen Gefüge vorliegen, umfassend die Schritte:
(i) Bereitstellen eines Stents, dessen Tragstruktur im expandierten Zustand vollständig in einem austenitischen Gefüge vorliegt; und
(ii) teilweises oder vollständiges Erwärmen ein oder mehrerer Tragstrukturabschnitte über eine Temperatur von 200°C, um eine Martensitbildung zu induzieren.

7. Verfahren nach Anspruch 6, bei dem das Erwärmen im Schritt (ii) mittels Laser- oder Elektronenstrahl, Plasma oder induktiv erfolgt.

8. Verfahren zur Herstellung eines Stents aus Nitinol mit einer Tragstruktur, die in Umfangsrichtung umlaufende Stege umfasst, die in axialer Richtung über Verbindungsstege miteinander verknüpft sind, und bei der die Tragstruktur einen komprimierten Zustand und einen expandierten Zustand einnehmen kann, wobei das Nitinol in der Tragstruktur im komprimierten Zustand in einem martensitischen Gefüge und im expandierten Zustand weitgehend in einem austenitischen Gefüge vorliegt, jedoch ein oder mehrere Tragstrukturabschnitte ganz oder in Teilen im expandierten Zustand in einem martensitischen Gefüge vorliegen, umfassend die Schritte:
(i) Bereitstellen eines Rohrs, das vollständig in einem austenitischen Gefüge vorliegt;
(ii) teilweises oder vollständiges Erwärmen ein oder mehrerer Rohrabschnitte über eine Temperatur von 200°C, um eine Martensitbildung zu induzieren; und
(iii) Herausschneiden der Tragstruktur des Stents aus dem Rohr.

9. Verfahren nach Anspruch 8, bei dem das Erwärmen im Schritt (ii) mittels Laser- oder Elektronenstrahl, Plasma oder induktiv erfolgt.
